# EUROPEAN PATENT APPLICATION

(11) **EP 2 368 884 A1**
(43) Date of publication of application: **28.09.2011**
(21) Application number: 10157824.3
(22) Date of filing: 25.03.2010
(51) Int. Cl.: C07D 239/69

(54) **Process for the preparation of bosentan**

(71) Applicant: Laboratorios Lesvi, S.L., 08970 Sant Joan Despi (ES)
(72) Inventor: Rodriguez, Sergio, 08970 Barcelona (ES); Huguet Clotet, Juan, 08970, Sant Joan Despi (ES)
(74) Representative: Ponti Sales, Adelaida

(57) **Abstract**

The present invention provides a novel process for obtaining Bosentan, with few synthesis steps, by coupling the intermediate pyrimidine compound of formula **I** with the sulfonamide compound of formula **II.** The use of said efficient process prevents the use of hazardous chemicals and thus it is of considerable interest for obtaining Bosentan in a large industrial scale. The invention also refers to the novel intermediates of formula **II** and to a process for its production.

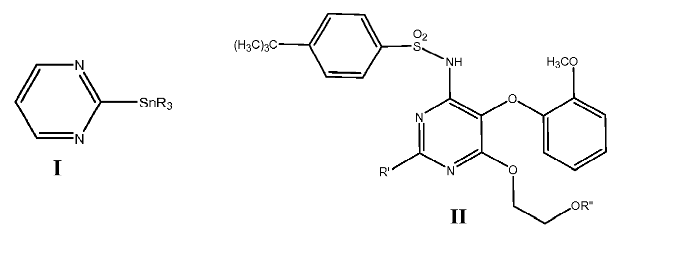

## Description

### FIELD OF THE INVENTION

The present invention relates to a novel process for the preparation of Bosentan or salts thereof and to intermediates of said process.

### BACKGROUND

Bosentan (**1**) or 4-tert-butyl-N-[6-(2-hydroxy-ethoxy)-5-(2-methoxy-phenoxy)-[2,2']-bipyrimidin-4-yl]-benzene-sulfonamide has a wide variety of biological activities. It is marketed as a monohydrate under the tradename Tracleer^{®}. Bosentan is an endothelin receptor antagonist indicated for the treatment of pulmonary arterial hypertension in patients with World Health Organization (WHO) class III or IV symptoms to improve exercise ability and decrease the rate of clinical worsening. Bosentan is useful in treatment of a variety of illnesses including cardiovascular disorders such as hypertension, ischemia, vasospasms and angina pectoris.

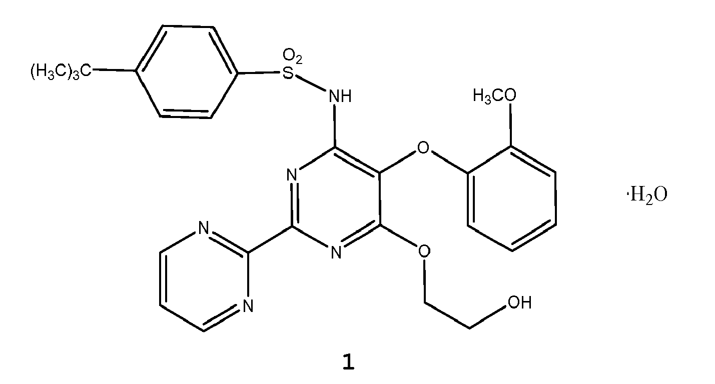

Bosentan was first disclosed by the European patent EP 0 526 708 B1 to Hoffmann La Roche. This patent describes the preparation of Bosentan through formation of the sulfonamide function through two different alternatives: a) condensation of a 4,6-dihalogenopyrimidine (**2**) and the appropriate benzenesulfonamide or b) condensation of a conveniently substituted amino-pyrimidine (**4**) and an appropriate benzenesulfohalogenide (see Scheme A). One of the major drawbacks of this synthesis is the use of ethylene glycol. As known, the use of ethylene glycol as a solvent is unworkable in industrial scale synthesis because of its toxicity and its high boiling point which requires a large amount of time and high energy consumption to remove it by distillation.

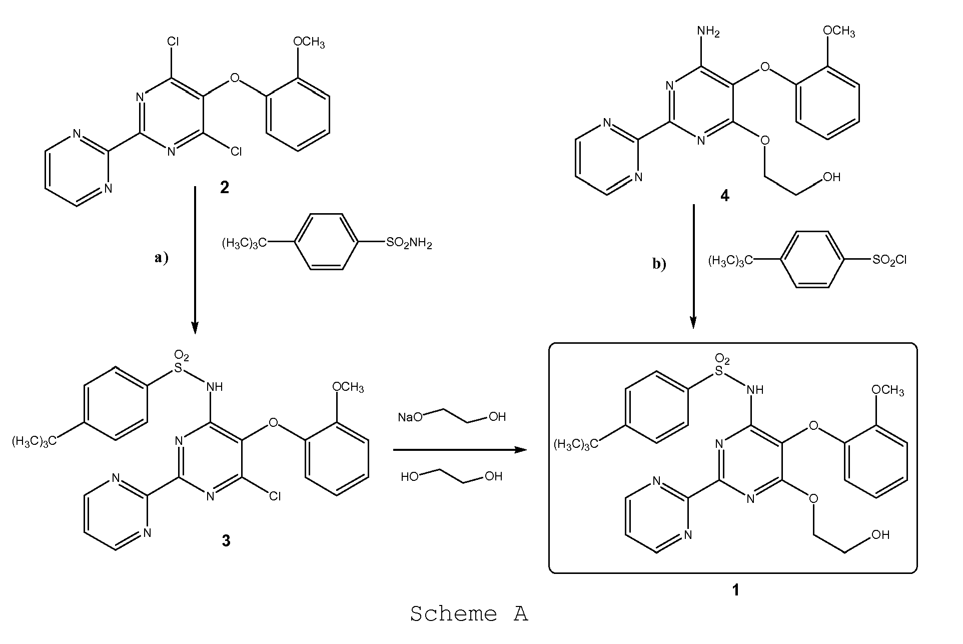

European patent EP 1 254 121 B1 provides an improved process for the preparation of Bosentan which avoids the use of ethylene glycol. Moreover, this novel process avoids the formation of undesired ethylene glycol bis-sulfonamide in which two molecules of the pyrimidine monohalide (**3**) are coupled with one molecule of ethylene glycol. The formation of bis-sulfonamide compounds requires costly and laborious separation steps to isolate a pharmaceutically suitable pure ethylene glycol sulfonamide compound. EP 1 254 121 B1 prepares a mono-protected 1,2-diheteroethylene substituted sulfonamide of the formula (**5**), which is isolated by recrystallization. The removal of the tert-butyl hydroxyl protective group by means of formic acid produces the 2-(formyloxy)ethoxy derivative (**6**), which after being concentrated and dried is treated with NaOH to yield Bosentan. Example 5 and 7 of EP 1 254 121 B1 describe the preparation of Bosentan through isolation of ethanol solvate derivative (**7**). The described process involves the use of protected reagents and deprotection steps, thus increasing the cost of the final product. See scheme B below.

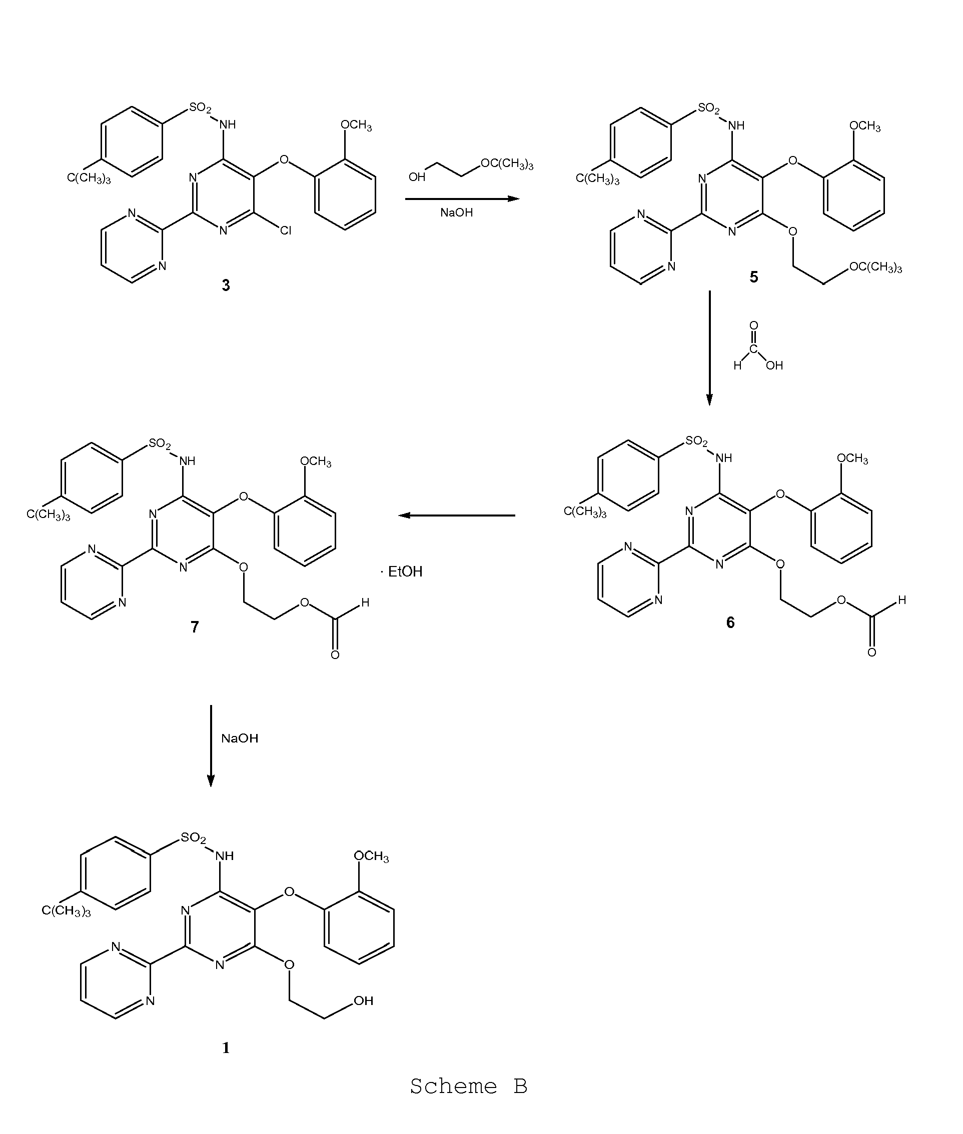

PCT patent application WO 2010/012637 provides a novel process for the preparation of Bosentan which comprises the coupling between a pyrimidine derivative and a sulfonamide derivative. In particular, the process requires the preparation of the sulfonamide derivative through diazotization of a primary amine and replacement of the diazonium group by a halide (Sandmeyer reaction). The resulting product is coupled with the pyrimidine compound. This process is depicted below in Scheme C.

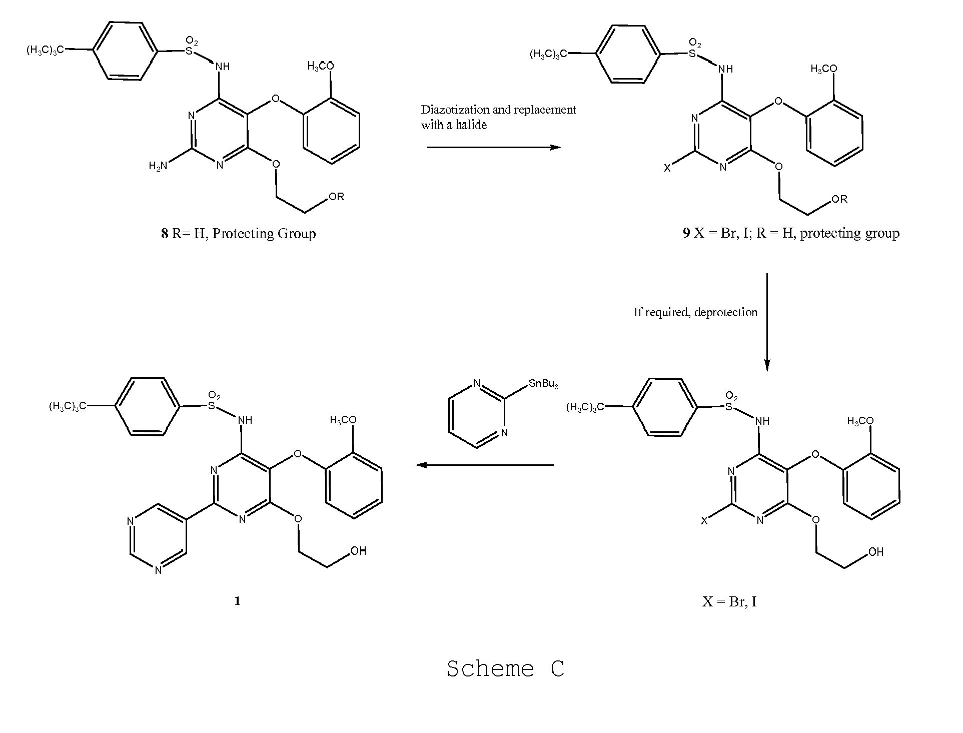

The preparation of compound (**9**), wherein X = Br as well as wherein X = I, provides the resulting product with low yields (45 % or below). These low yields increase the cost of the final product and, as a consequence, the overall process is not desirable at industrial scale. Not only low yields are not desirable but also the use of reactants that present toxic effects on human health and which are environmental poisoning.

Therefore, there is a need for an improved process for the preparation of Bosentan which overcomes the above mentioned problems.

### BRIEF DESCRIPTION OF THE INVENTION

The aim of the present invention is to provide an efficient and safe process for the preparation of Bosentan and salts thereof which can be applied at an industrial level with low energy and costs and high yields.

The authors of the present invention have found that Bosentan can advantageously be prepared in high yield avoiding the use of intermediate compounds of formula (**9**) which are difficult to obtain from amino derivative compounds of formula (**8**). The process of the invention does not require the intermediate amino compound (**8**) avoiding the undesirable azide chemistry associated with its production. The process of the invention also avoids the use of undesired toxic reactants such as nitrites and diiodomethane.

Accordingly, a first aspect of the present invention is a process for the preparation of Bosentan or a pharmaceutically salt thereof which comprises a coupling reaction between a pyrimidine compound of formula **I**: 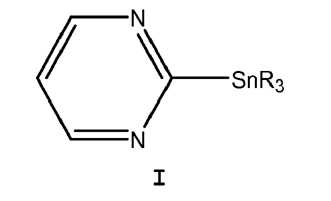 wherein R is a linear or branched C₁-C₅ alkyl and a sulfonamide compound of formula **II** 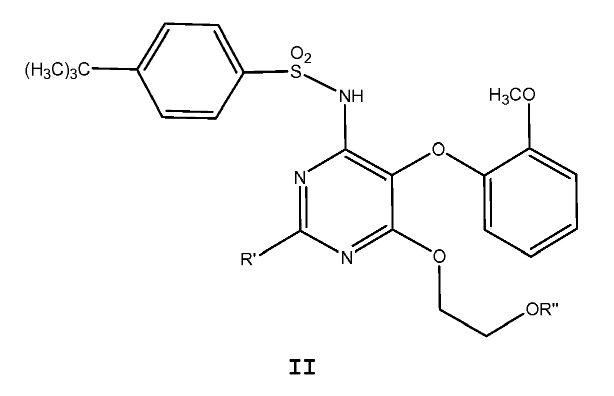 wherein R' is a sulfonyloxy group and R" is selected from hydrogen, a sulfonyl group and a protective groups of alcohols, in the presence of a palladium catalyst and in a solvent medium and, if required, removing of the sulfonyl group or the protective group of alcohols.

A second aspect of the present invention relies on sulfonamide compounds of formula **II** or salts thereof.

A third aspect of the present invention is a process for the preparation of the novel sulfonamide compounds of formula **II** above.

A fourth aspect of the present invention relies on sulfonamide compounds of formula **IV** or salts thereof.

A fifth aspect of the present invention is the use of the aforementioned sulfonamide compound of formula **II** or its salts for the preparation of Bosentan.

A sixth aspect of the present invention is the use of the aforementioned sulfonamide compound of formula **IV** or its salts for the preparation of Bosentan.

### BRIEF DESCRIPTION OF THE FIGURES

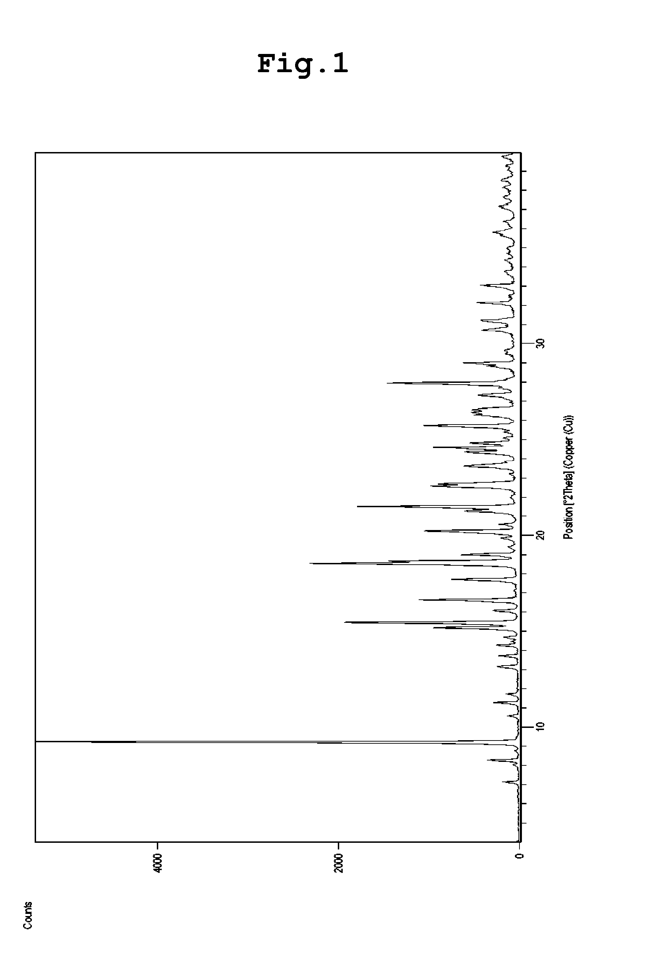

Figure 1 shows the X-Ray Diffraction Pattern of the Bosentan monohydrate obtained according to the process of the present invention, and then purified. The purifying method was carried out according to the example 8 of EP 1 254 121 B1.

### DETAILED DESCRIPTION OF THE INVENTION

In the context of the present invention, the following terms have the meaning detailed below:
The term "palladium catalyst" is taken to mean a compound of palladium, which can be homogeneous and soluble in the reaction medium, such as Pd(PPh₃)₄, PdCl₂ (PPh₃) ₂, Pd(AcO)₂, Pd₂(dba)₃, PdCl₂ (dppf), PdCl₂(CH₃CN)₂, Pd (P^{t}Bu₃)₂ wherein AcO is acetate, Ph is phenyl, dba is dibenzylideneacetone, dppf is 1,1'-bis(diphenylphosphino)ferrocene, and ^{t}Bu is tert-butyl or which can be heterogeneous and insoluble in the reaction medium, such as Pd/C.

The term "ligand" is taken to mean an organic compound of the phosphine or amine type capable of coordinating and activating the palladium species which catalyses the desired reaction, such as triphenylphosphine and tri-tert-butylphosphine.

The term "linear or branched C₁-C₅ alkyl" relates to a linear or branched hydrocarbon radical consisting of carbon and hydrogen atoms, which does not contain unsaturation, having one to five carbon atoms and which is joined to the rest of the molecule by a single bond. Examples of alkyl groups include methyl, ethyl, propyl, isopropyl, butyl, isobutyl, tert-butyl, sec-butyl and pentyl. Preferably, the alkyl used is butyl.

In this invention a "protective group of alcohols" should be understood broadly. Examples of protective group of alcohols can be found in "Protective Groups in Organic Synthesis, 3rd edition, T.W. Greene, Wiley Interscience, chapter 2". Preferably, the protective group of alcohols is of ether type such as methyl-, ethyl-, propyl-, butyl-, benzyl- trityl-ether, all of them optionally substituted by alkyl or aryl radicals. Also preferably, a protective group of alcohols according to the invention is of silyl ether type such as trimethylsilyl- (TMS), tert-butyldiphenylsilyl-(TBDPS) , triisopropylsilyl- (TIPS), [2-(trimethylsilyl)ethoxy]methyl- (SEM) ether. Also preferably are protective groups of alcohols of ester type such as acetyl-, benzoyl-, pivaloyl-ester, etc.

As "sulfonyloxy group" it is understood a substituted or unsubstituted alkyl or aryl sulfonyloxy group. Examples of sulfonyloxy groups used in this invention are mesylate (methanesulfonate), tresylate (2,2,2-trifluoroethanesulfonate), tosylate (4-toluensulfonate), brosylate (p-bromobenzenesulfonate), nosylate (4-nitrobenzenesulfonate), triflate (trifluoromethanesulfonate), nonaflate (nonafluorobutanesulfonate), tridecafluoroheptanesulfonate, heptadecafluorooctanesulfonate and fluorosulfonate. Preferably, a sulfonyloxy group is triflate.

By the term "sulfonyl group" it is understood a substituted or unsubstituted alkyl or aryl sulfonyl group. Examples of sulfonyl goups used in this invention are mesyl (methanesulfonyl), tresyl ((2,2,2-trifluoroethanesulfonyl), tosyl (4-toluensulfonyl), brosyl (p-bromobenzenesulfonyl), nosyl (4-nitrobenzenesulfonyl), trifluoromethanesulfonyl, nonafluorobutanesulfonyl, tridecafluoroheptanesulfonyl, heptadecafluorooctanesulfonyl and fluorosulfonyl.

According to a first aspect, the present invention is directed to a process for the preparation of Bosentan or a pharmaceutically salt thereof which comprises a coupling reaction between a pyrimidine compound of formula **I**: 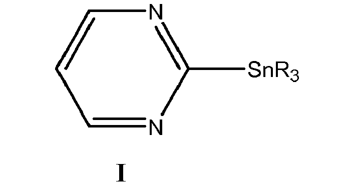 wherein R is a linear or branched C₁-C₅ alkyl and a sulfonamide compound of formula **II** 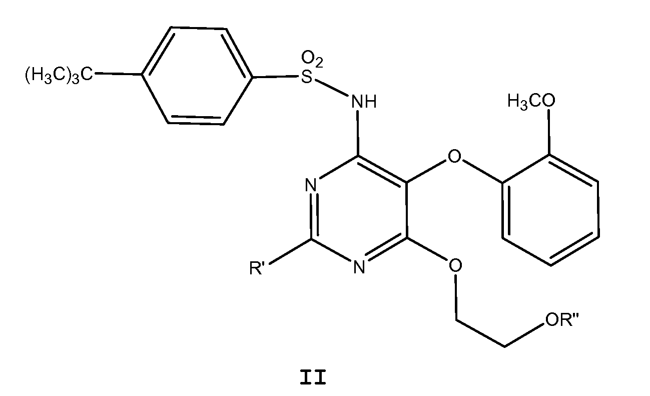 wherein R' is a sulfonyloxy group and R" is selected from hydrogen, a sulfonylgroup and a protective groups of alcohols, in the presence of a palladium catalyst and in a solvent medium and, if required, removing the sulfonyl group or the protective group of alcohols.

The use of compounds of formula **II** in the coupling reaction of the present invention avoids the preparation of compounds (**8**) and (**9**) (see Scheme C). Thus, no diiodomethane and/or nitrites, which are required in the preparation of compound (**9**), are employed. These reagents are highly environmental toxic and extremely unhealthy. Moreover, they are difficult to eliminate from the final product.

In addition, the process of the invention does not need the preparation of compound (**8**). The introduction of the amino group in compound (**8**) requires the use of azide followed by hydrogenation. As known, when an azide reaction is considered for production in pilot-plant or with manufacturing scale equipment, several safety concerns must be addressed. Of great concern is the safety issues associated with hydrazoic acid. The potential hazard associated with hydrazoic acid in the reactor headspace is one of the main barriers to use the azide chemistry on large scale.

The inventors have discovered that the coupling reaction between the pyrimidines of formula **I** and the sulfonamides of formula **II** can proceed with and without the need to protect the terminal alcohol moiety of the compound of formula **II.** On the one hand, the presence of a protective group or a sulfonyl group R" in compounds of formula **II** facilitates the purification of the final product. On the other, when R" is hydrogen there is no need of an extra step for removing the protective group.

It should be pointed out that the sulfonyloxy group R' and -O-R" in compound of formula **II** can be the same or different.

The coupling reaction between said compounds **I** and **II** is performed by the use of a palladium catalyst. It will be immediately apparent to the skilled person that a wide range of palladium based catalysts are suitable for this reaction. The hetereoaromatic coupling between aromatic trialkyl tin derivatives and aromatic electrophiles is commonly known by those skilled in the art as Stille reaction.

The reaction takes place in the presence of a palladium catalyst and in a solvent medium and, if required, followed by the elimination of the protective group of alcohols. The palladium based catalyst is used in less than stoichiometric amounts for economic and environmental reasons, but it is readily obvious to the skilled person that the process of the invention will also proceed by adding stoichiometric amounts of the palladium based catalyst or excess of the same.

The reaction may proceed in homogeneous and heterogeneous phase. Preferably, the reaction proceeds in homogenous phase.

When the reaction is performed under homogeneous conditions, preferred catalysts are selected from the group consisting of Pd (PPh₃)₄, PdCl₂(PPh₃)₂, Pd(AcO)₂, Pd₂(dba)₃, PdCl₂(dppf), PdCl₂(CH₃CN)₂ and Pd[P^{t}Bu₃]₂.

According to a preferred embodiment, the process of the invention is carried out in the presence of a ligand, being said ligand preferably phosphines when Pd(AcO)₂ and Pd₂(dba)₃ are used. Many phosphines may be found in the art. The election of the most suitable phosphine is a matter of routine experimentation for the skilled person and fine-tunes parameters of the reaction such as the yield, the speed or the turn over of the catalyst. According to a preferred embodiment, the reaction is carried out in the presence of triphenylphosphine or tri-tert-butylphosphine.

The reaction may proceed in the presence of a solvent medium which comprises organic solvents and/or water. For example, polar solvents such as N,N-dimethylformamide (DMF), acetonitrile, dimethylacetamide (DMA), N-methylpyrrolidone (NMP), ethers such as 1,2-dimethoxyethane (DME), diethoxymethane (DEM), tetrahydrofuran (THF), dioxane; aromatic solvents such as toluene or xylene; or alcohols like methanol, ethanol, propanol or mixtures thereof. Election of the most suitable solvent requires only routine experimentation for the skilled person.

Optionally, the process of the invention may be carried out in the presence of organic bases such as tertiary amines, like triethyl amine (TEA) or N,N-diisopropylethylamine (DIPEA), or other ammonia derivatives in which one or more hydrogen have been substituted by alkyl or aryl radicals such as ethyl, isopropyl, benzyl, phenyl or pyridine. The process of the invention may be also carried out in the presence of inorganic salts such chlorides (e.g. lithium chloride, sodium chloride, potassium chloride); or phosphates of alkaline or alkaline earth metals (e.g. potassium phosphate); or fluorides (potassium fluoride, cesium fluoride). Preferably, organic bases are used.

Preferably, the reaction temperature is comprised between 25°C and 200°C and when the reaction is finished, it is usually followed by purifying methods known to the skilled person (e.g. chromatography or HPLC). Normal reaction times are between 1 and 48 hours, preferably under inert atmosphere.

Pyrimidine compounds of formula **I** may be prepared by methods known in the art. For example, see the experimental section of J. Org. Chem. 1995, 60, 3020-3027, CA 2071193 and WO 2007058602.

A second aspect of the invention is directed to sulfonamide compounds of formula **II** or salts thereof: 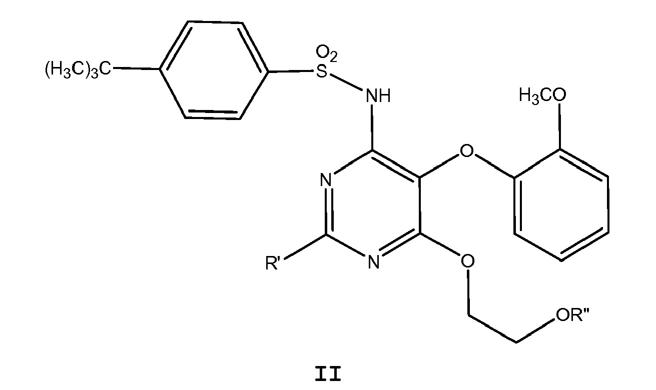 wherein R' is a sulfonyloxy group and R" is selected from hydrogen, a sulfonyl group and a protective groups of alcohols.

Said compounds of formula **II** are novel intermediates in the process of the invention. R' in compounds of formula **II** is a substituted or unsubstituted alkyl or aryl sulfonyloxy group. Examples of sulfonyloxy groups R' or -O-R" in compounds of formula **II** are mesylate, tresylate, tosylate, brosylate, nosylate, triflate, nonaflate, tridecafluoroheptanesulfonate, heptadecafluorooctanesulfonate and fluorosulfonate. Preferably, a sulfonyloxy group is triflate. Both sulfonyloxy groups R' and -O-R" can be the same or different. Protective groups of alcohols defined as R" in compounds of formula **II** are of ether type such as methyl-, ethyl-, propyl-, butyl-, benzyl- trityl-ether, all of them optionally substituted by alkyl or aryl radicals. Also preferably, a protective group of alcohols according to the invention is of silyl ether type such as trimethylsilyl- (TMS), tert-butyldiphenylsilyl- (TBDPS), triisopropylsilyl- (TIPS), [2-(trimethylsilyl)ethoxy]methyl- (SEM) ether. Other protective groups of alcohols are of ester type such as acetyl-, benzoyl-, pivaloy-ester, etc.

It will be appreciated, that in a particular embodiment, the preparation of Bosentan is advantageously performed when R' and R" in compounds of formula **II** are the same because the transformation of the hydroxyl group of compound **IV** and the protection terminal alcohol can be done in the same reaction step. Preferably, R' and -O-R" are triflate. In view of definitions above, it is readily obvious to the skilled person that R' is a sulfonyloxy group and R" is a sulfonyl group, but the moiety -O-R" is also understood as a sulfonyloxy group.

The compounds of formula **II** may be obtained from intermediate of formula **IV:** 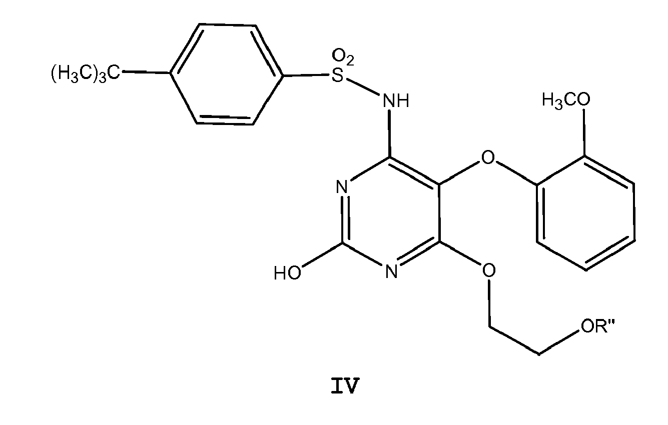 wherein R" is selected from hydrogen, a sulfonyl group and a protective group of alcohols.

Said compounds of formula **IV** are novel intermediates in the process of the invention. Sulfonyloxy groups defined as -O-R" in compounds of formula **IV** are mesylate, tresylate, tosylate, brosylate, nosylate, triflate, nonaflate, tridecafluoroheptanesulfonate, heptadecafluorooctanesulfonate and fluorosulfonate. Protective groups of alcohols defined as R" in compounds of formula **IV** are of ether type such as methyl-, ethyl-, propyl-, butyl-, benzyl- trityl-ether, all of them optionally substituted by alkyl or aryl radicals. Other protective group of alcohols according to the invention is of silyl ether type such as trimethylsilyl- (TMS), tert-butyldiphenylsilyl- (TBDPS), triisopropylsilyl- (TIPS), [2-(trimethylsilyl)ethoxy]methyl- (SEM) ether. Other protective groups of alcohols are of ester type such as acetyl-, benzoyl-, pivaloy-ester, etc.

Another aspect of the invention relates to a process for the preparation of a sulfonamide compound of formula **II** which comprises the following steps:
a) replacement of the sulfone group of compound of formula **III** by an hydroxyl group to yield a compound of formula **IV** 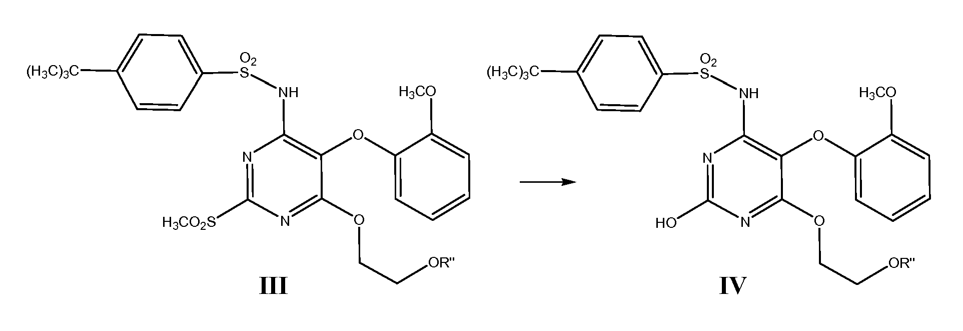 wherein R" is selected from hydrogen, a sulfonyl group or a protective group of alcohols
b) transformation of the aromatic hydroxyl group of compound **IV** to a substituted or unsubstituted alkyl or aryl sulfonyloxy group, R', and,
c) if required, removing the sulfonyl group or the protective group of alcohols, R", of compound of formula **IV**
   Step a) of the process requires the use of sodium hydroxide which is added to a solution of the sulfonamide compound of formula **III** in an organic solvent at 25-110°C. Suitable organic solvents are THF, 2-Methyltetrahdyrofuran, acetonitrile, dioxane, DMF, DMA, NMP, dimethyl sulfoxide (DMSO), methyl ethyl ketone (MEK), Methyl isobutyl ketone (MIBK) and alcohols (methanol, ethanol, butanol, isopropanol, and the like) or mixtures thereof.
   Step b) of the process comprises the transformation of the hydroxyl group to a substituted or unsubstituted alkyl or aryl sulfonyloxy group. Examples of sulfonyloxy groups that can be used are mesylate, tresylate, tosylate, brosylate, nosylate, triflate, nonaflate, tridecafluoroheptanesulfonate, heptadecafluorooctanesulfonate and fluorosulfonate. Among the reactants that can be used to perform this replacement and which are well known by the skilled man in the art are the corresponding anhydrides or acid halides. For example, if R' in compound of formula **II** is triflate, then the reactant used in step b) should be triflic anhydride or triflic chloride. This reaction requires the dissolution of the hydroxyl compound of formula **IV** in an organic solvent such as dichloromethane, DMF, DMA, THF, dioxane, DEM, DME, pyridine and mixtures thereof. Optionally, when a non basic solvent is used, an organic base should be added. Organic bases that can be used are tertiary amines, like TEA or DIPEA, or alkaline or alkaline-earth acetates (e.g. sodium acetate); or other ammonia compounds in which one or more hydrogen have been substituted by alkyl or aryl radicals such as ethyl, isopropyl, benzyl, phenyl or pyridine. The reaction temperature is kept between -50 and 25°C.
   Step c) of the process, if required, comprises the elimination of the sulfonyl group or the elimination of the protective group, R", which is carried out by conventional methods.

Sulfonamide compounds of formula **III** can be prepared by methods known in the art. For example see Bioorg. Med. Chem. Lett. 2002, 12, 81-84.

Bosentan obtained according to the process of the present invention can be milled or micronised to obtain a D₅₀ and D₉₀ particle size of less than about 400 µm, preferably less than about 200 µm, more preferably less than about 150 µm, and most preferably less than about 60 µm. It is noted the notation Dₓ means that X% of the particles in a composition have a diameter less than a specified diameter D. Thus, a D₅₀ of about 400 µm means that 50% of the micronised bosentan particles have a diameter less than 400 µm.

Particles of this size are obtained by conventional methods, e.g. grinding in an air jet mill, hammer and screen mill, fine impact mill, ball mill or vibrator mill.

Micronisation is preferably effected by known methods using an ultrasonic disintegrator or by stirring a suspension with a high speed agitator.

The present invention is illustrated in more detail by the following Examples but should not be construed to be limited thereto.

### EXAMPLES

### Example 1

***N*-[6-(2-*tert*-Butoxyethoxy)-2-hydroxy-5-(2-methoxy-phenoxy)-pyrimidin-4-yl]-4-*tert-*butylbenzenesulfonamide (11)**

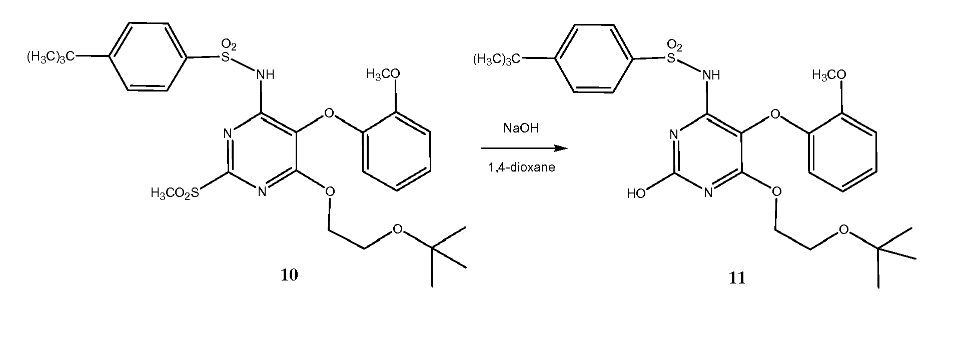

To a solution of compound (**10**) (1g, 1.64 mmol) in 1,4-dioxane (5 mL) was added a 1 M solution of sodium hydroxide (5 mL). The reaction was heated at 100°C for 1 h and then, the mixture was cooled to room temperature. The pH was adjusted to 3-4 with HCl 3M at 0°C, and methylene chloride was added. The organic phase was separated, dried and evaporated. The residue obtained was purified by column chromatography on silica gel to afford pure compound (**11**) (802 mg, 89 % yield) as a slightly yellow solid.

**¹H-RMN (200 MHz, CDCl₃)** : 1.23 (s, 9H); 1.31 (s, 9H); 3.67 (m, 2H); 3.94 (s, 3H); 4.43 (m, 2H); 4.75 (bs, 2H); 6.81-7.18 (m, 4H), 7.39 (d, J = 8.0 Hz, 2H); 7.65 (d, J = 8.0 Hz, 2H) ppm.

### Example 2

**Trifluoromethanesulfonic acid 4-(2-*tert*-butoxyethoxy)-6-(4-*tert*-butylbenzenesulfonylamino)-5-(2-methoxyphenoxy)-pyrimidin-2-yl ester (12)**

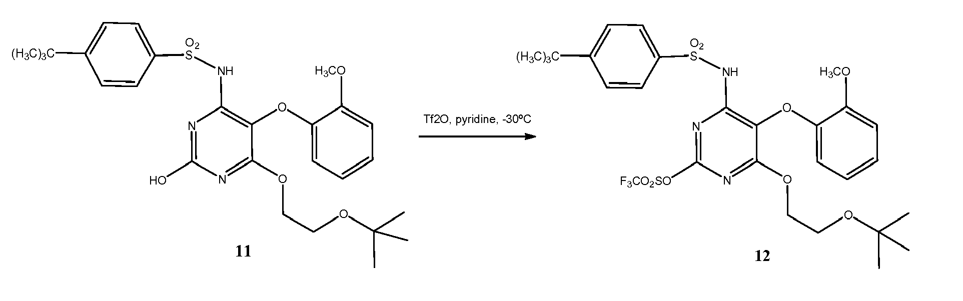

To a solution of compound (**11**) (1.4 g, 2.6 mmol) in dry pyridine (14 mL), cooled to -30 °C and under nitrogen atmosphere, was slowly added triflic anhydride (530 µL, 3.1 mmol). After 1 h at this temperature, the reaction mixture was added over CHCl₂ / H₂O, the pH adjusted to 3-3.5 with 3 M HCl and the organic phase separated and dried. Evaporation of solvent and purification of the residue by column chromatography over silica gel afforded the corresponding aryl triflate (**12**) (1.5 g, 85 % yield) as a yellowish solid.

**¹H-RMN (200 MHz, CDCl₃)** : 1.17 (s, 9H); 1.32 (s, 9H); 3.65 (m, 2H); 4.04 (s, 3H); 4.45 (m, 2H); 6.85-7.21 (m, 4H); 7.52 (d, J = 8.0 Hz, 2H); 8.02 (d, J = 8.0 Hz, 2H); 9.61 (s, 1H) ppm.

### Example 3

**Trifluoromethanesulfonic acid 4-(4-tert-butylbenzenesulfonylamino)-6-(2-hydroxyethoxy)-5-(2-methoxyphenoxy)-pyrimidin-2-yl ester (13)**

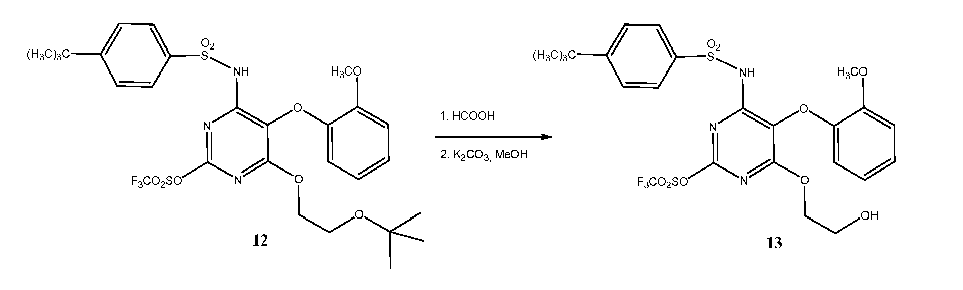

Compound (**12**) (1.4 g, 2.0 mmol) in 98 % HCOOH (4.2 mL) was heated at 85 °C and the reaction mixture stirred for 90 min. The reaction was the cooled at room temperature and was added over toluene / H₂O. The bottom layer was cut and then back-extracted twice with toluene. The combined organic extracts were dried and evaporated. The residue was dissolved in 13 mL of MeOH and cooled down to 0°C. Solid K₂CO₃ (715 mg, 5.2 mmol) was charged and the reaction mixture was aged at that temperature for 1 h. Then, the mixture was allowed to reach 20 °C and EtOAc and H₂O were added. The pH was adjusted to 3-3.5 with 3 M HCl and the organic layer was separated and dried. After solvent was evaporated, the residue thus obtained was purified by column chromatography over silica gel affording the corresponding deprotected aryl triflate (**13**) (1.1 g, 86 % yield) as a white solid.

**¹H-RMN (200 MHz, CDCl₃)** : 1.35 (s, 9H); 3.78 (m, 2H); 3.92 (s, 3H); 4.38 (m, 2H); 6.82-7.22 (m, 4H); 7.54 (d, J = 8.0 Hz, 2H); 8.05 (d, J = 8.0 Hz, 2H); 8.67 (s, 1H) ppm.

### Example 4

**4-*tert*-butyl-*N*-[6-(2-hydroxyethoxy)-5-(2-methoxy-phenoxy)-[2,2']bipyrimidinyl-4-yl]-benzenesulfonamide (Bosentan)**

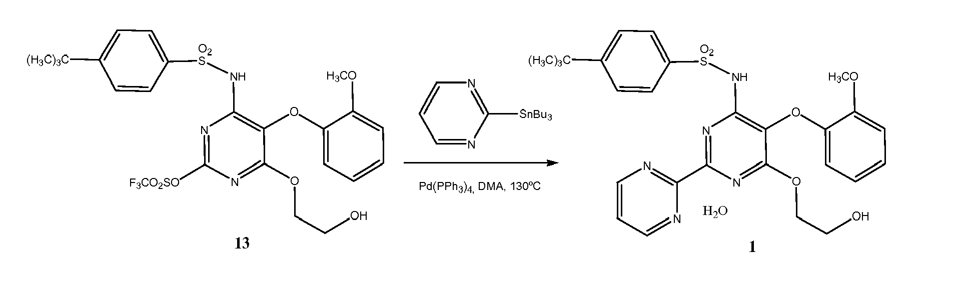

A solution of 2-tributylstannanyl pyrimidine (390 mg, 1.05 mmol), 2-pyrimidin triflate intermediate (**13**) (510 mg, 0.81 mmol), and Pd(PPh₃)₄ (95 mg, 0.081 mmol) in dry dimethylacetamide (7.5 mL) was heated at 130°C for 6 h. After being cooled to room temperature, the reaction was filtered through a plug of Celite^{™}, the filtrate was diluted with water and extracted twice with ethyl acetate. The combined organic layers were washed with brine, solvent was removed and the residue was purified by silica gel chromatography furnishing the desired compound as a pale yellow solid (357 mg, 79 %). The crude bosentan was further purified by recrystallization in hot H₂O / EtOH affording pure bosentan monohydrate (**1**) as a white solid (327 mg, 70 %).

**¹H-RMN (200 MHz, d₆-DMSO)**: 1.29 (s, 9H); 3.85 (m, 2H); 3.93 (s, 3H); 4.59 (m, 2H); 6.81-7.18 (m, 4H), 7.43 (d, J = 8.0 Hz, 3H); 8.45 (d, J = 8.0 Hz, 2H); 9.01 (s, 2H) ppm.

**DSC-TG**: endothermic peak at 116.53 °C, with a loss of weight of 3.1 % (monohydrate).

D₁₀ : 1.36 µm, D₅₀ : 20.32 µm, D₉₀ : 56.64 µm

### Example 5

**N-[6-(2-tert-Butoxy-ethoxy)-5-(2-methoxy-phenoxy)-[2,2']bipyrimidinyl-4-yl]-4-ethyl-benzenesulfonamide (14)**

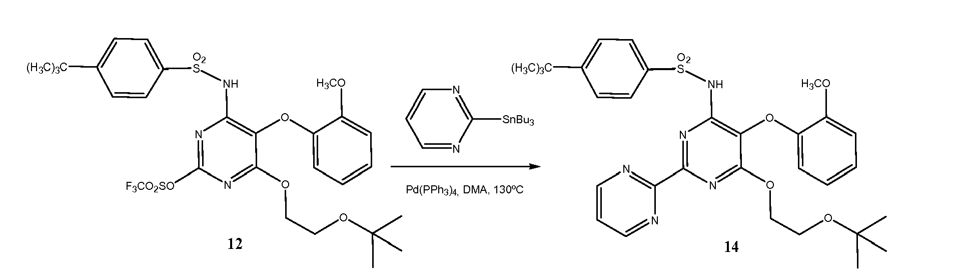

A solution of 2-tributylstannanyl pyrimidine (212 mg, 0.57 mmol), 2-pyrimidin triflate intermediate (**12**) (300 mg, 0.44 mmol), and Pd(PPh₃)₄ (52 mg, 0.044 mmol) in dry dimethylacetamide (4.5 mL) was heated at 130°C for 6 h. After being cooled to room temperature, the reaction was filtered through a plug of Celite^{™}, the filtrate was diluted with water and extracted twice with ethyl acetate. The combined organic layers were washed with brine, solvent was removed and the residue was purified by silica gel chromatography furnishing the desired compound as a pale yellow solid (216 mg, 80 %).

**¹H-RMN (200 MHz, CDCl₃)** : 1.13 (s, 9H); 1.26 (s, 9H); 3.62 (m, 2H); 4.00 (s, 3H); 4.63 (m, 2H); 6.81-7.18 (m, 4H), 7.43 (m, 3H); 8.23 (d, J = 8.0 Hz, 2H); 9.01 (s, 2H) ppm.

### Example 6

**4-tert-butyl-N-[6-(2-hydroxyethoxy)-5-(2-methoxy-phenoxy)-[2,2']bipyrimidinyl-4-yl]-benzenesulfonamide (Bosentan 1)**

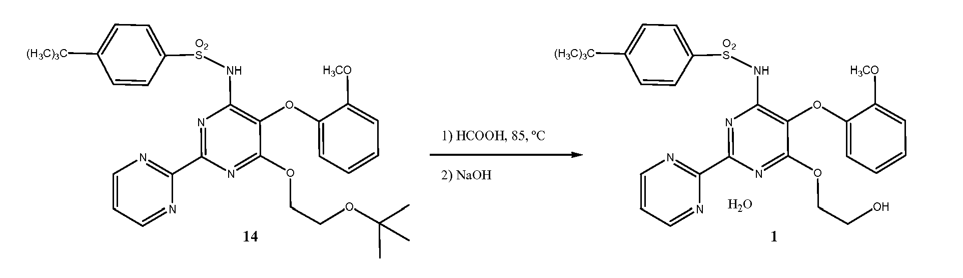

Compound (**14**) (1.0 g, 1.64 mmol) in 98 % HCOOH (3.0 mL) was heated at 85 °C and the reaction mixture stirred for 90 min. The reaction was then cooled at room temperature and was added over CH₂Cl₂ / H₂O. The bottom layer was cut and then back-extracted twice with CH₂Cl_{2.} The combined organic extracts were dried and evaporated. The residue was dissolved in 10 mL of MeOH and then solid K₂CO₃ (715 mg, 5.2 mmol) was charged. The reaction mixture was aged at that temperature for 1 h. Then, EtOAc and H₂O were added, the pH was adjusted to 3-3.5 with 3 M HCl and the organic layer was separated and dried. After solvent was evaporated, the residue thus obtained was purified by column chromatography over silica gel affording the corresponding bosentan (**1**) (780 mg, 86 % yield) as a yellowish solid. The crude bosentan was further purified by recrystallization in hot H₂O / EtOH affording pure bosentan monohydrate (**1**) as a white solid (655 mg, 70 %).

**¹H-RMN (200 MHz, d₆-DMSO)** : 1.29 (s, 9H); 3.85 (m, 2H); 3.93 (s, 3H); 4.59 (m, 2H); 6.81-7.18 (m, 4H), 7.43 (d, J = 8.0 Hz, 3H); 8.45 (d, J = 8.0 Hz, 2H); 9.01 (s, 2H) ppm.

### Example 7

**4-tert-Butyl-N-[2-hydroxy-6-(2-hydroxy-ethoxy)-5-(2-methoxy-phenoxy)-pyrimidin-4-yl]-benzenesulfonamide (15)**

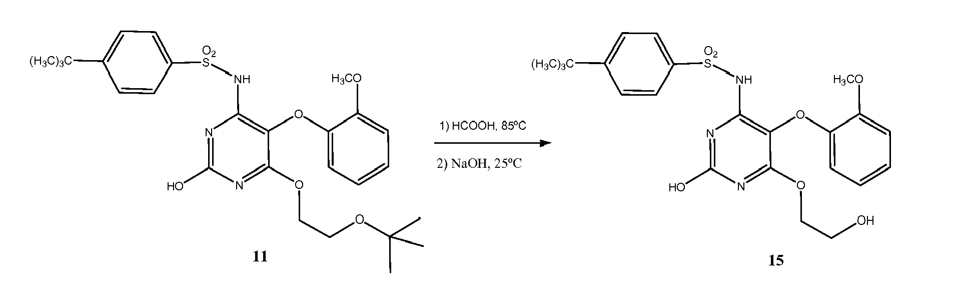

Compound (**11**) (1.0 g, 1.83 mmol) in 98 % HCOOH (3.0 mL) was heated at 85 °C and the reaction mixture stirred for 120 min. The reaction was the cooled at room temperature and was added over CH₂Cl₂ / H₂O. The bottom layer was cut and then back-extracted twice with CH₂Cl_{2.} The combined organic extracts were dried and evaporated. The residue was dissolved in 10 mL of MeOH and then solid NaOH (300 mg, 7.32 mmol) was charged. The reaction mixture was aged at that temperature for 1 h. Then, EtOAc and H₂O were added, the pH was adjusted to 3-3.5 with 3 M HCl and the organic layer was separated and dried. After solvent was evaporated, the residue thus obtained was purified by column chromatography over silica gel affording the corresponding diol (**7**) (753 mg, 84 % yield) as a white-off solid.

**¹H-RMN (200 MHz, d₆-DMSO)** 1.29 (s, 9H); 3.85 (m, 2H); 3.93 (s, 3H); 4.59 (m, 2H); 6.81-7.18 (m, 4H), 7.43 (d, J = 8.0 Hz, 2H); 8.45 (d, J = 8.0 Hz, 2H) ppm.

### Example 8

**4-tert-Butyl-N-[2-hydroxy-6-(2-hydroxy-ethoxy)-5-(2-methoxy-phenoxy)-pyrimidin-4-yl]-benzenesulfonamide (15)**

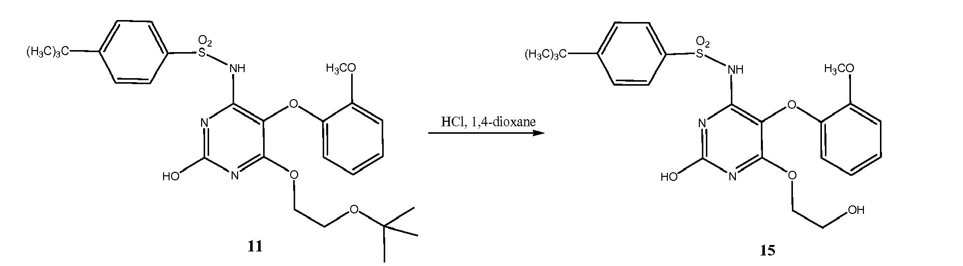

Compound (**11**) (1.0 g, 1.83 mmol) was treated with 10 ml of a 4 N solution of HCl in dioxane at room temperature. The reaction was stirred for 2 h and then added over CH₂Cl₂ / H₂O. The pH was adjusted to 3.0-3.5 with 1 N solution of sodium hydroxide, the bottom layer was cut and then back-extracted twice with CH₂Cl₂. The combined organic extracts were dried and evaporated and the residue thus obtained was purified by column chromatography over silica gel affording the corresponding diol (**15**) (753 mg, 84 % yield) as a white solid.

### Example 9

**Trifluoromethanesulfonic acid 4-(4-tert-butylbenzenesulfonylamino)-6-(2-hydroxyethoxy)-5-(2-methoxyphenoxy)-pyrimidin-2-yl ester (13)**

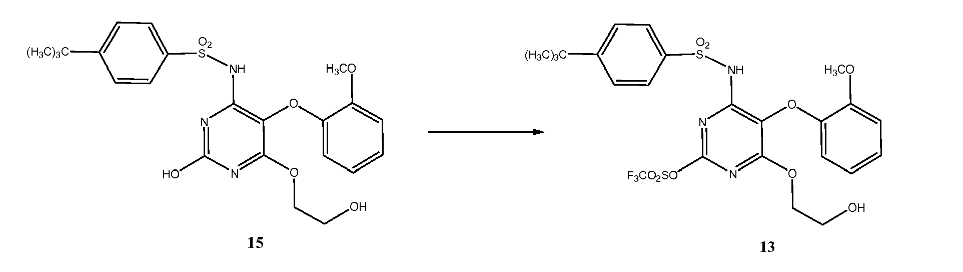

To a solution of compound (**15**) (1.4 g, 2.86 mmol) in dry pyridine (14 mL), cooled to -30 °C and under nitrogen atmosphere, was slowly added triflic anhydride (480 µL, 2.86 mmol). After 1 h at this temperature, the reaction mixture was added over CHCl₂ / H₂O, the pH adjusted to 3-3.5 with 3 M HCl and the organic phase separated and dried. Evaporation of solvent and purification of the residue by column chromatography over silica gel afforded the corresponding aryl triflate (**13**) (1.42 g, 80 % yield) as an off-white solid.

**¹H-RMN (200 MHz, CDCl₃)** : 1.35 (s, 9H); 3.78 (m, 2H); 3.92 (s, 3H); 4.38 (m, 2H); 6.82-7.22 (m, 4H); 7.54 (d, J = 8.0 Hz, 2H); 8.05 (d, J = 8.0 Hz, 2H); 8.67 (s, 1H) ppm.

### Example 10

**Trifluoromethanesulfonic acid 2-[6-(4-tert-butyl-benzenesulfonylamino)-5-(2-methoxy-phenoxy)-2-trifluo-romethanesulfonyloxy-pyrimidin-4-yloxy]-ethyl ester (16)**

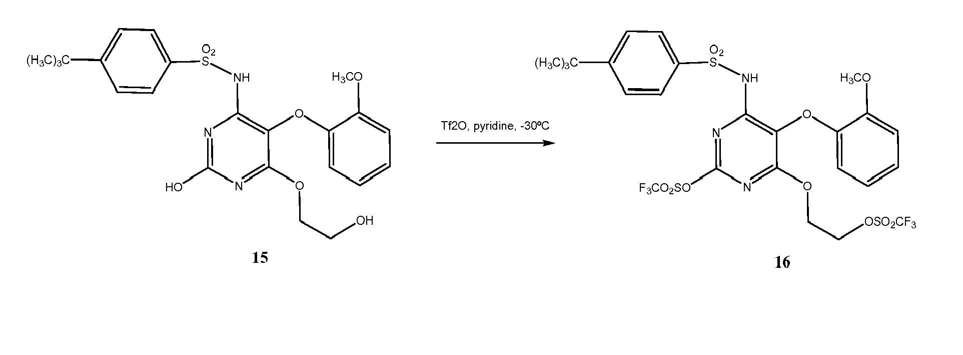

To a solution of compound (**15**) (2.0 g, 4.0 mmol) in dry pyridine (20 mL), cooled to -30 °C and under nitrogen atmosphere, was slowly added triflic anhydride (1.48 mL, 8.8 mmol). After 1 h at this temperature, the reaction mixture was added over CHCl₂ / H₂O, the pH adjusted to 3-3.5 with 3 M HCl and the organic phase separated and dried. Evaporation of solvent and purification of the residue by column chromatography over silica gel afforded the corresponding aryl triflate (**16**) (2.77 g, 92 % yield) as a slightly yellow solid.

**¹H-RMN (200 MHz, CDCl₃)** : 1.17 (s, 9H); 1.32 (s, 9H); 3.98 (m, 2H); 4.04 (s, 3H); 4.35 (m, 2H); 6.85-7.21 (m, 4H); 7.52 (d, J = 8.0 Hz, 2H); 8.02 (d, J = 8.0 Hz, 2H); 9.61 (s, 1H) ppm.

### Example 11

**N-[6-(2-tert-Butoxy-ethoxy)-5-(2-methoxy-phenoxy)-[2,2']bipyrimidinyl-4-yl]-4-ethyl-benzenesulfonamide (17)**

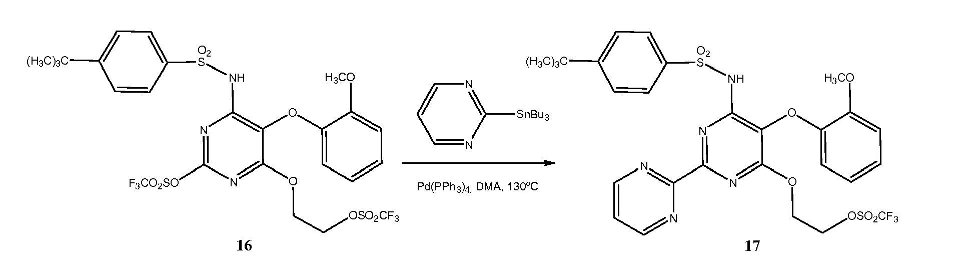

A solution of 2-tributylstannanyl pyrimidine (294 mg, 0.79 mmol), 2-pyrimidin triflate intermediate (**16**) (500 mg, 0.66 mmol), and Pd(PPh₃)₄ (76 mg, 0.066 mmol) in dry dimethylacetamide (7.5 mL) was heated at 130°C for 6 h. After being cooled to room temperature, the reaction was filtered through a plug of Celite^{™}, the filtrate was diluted with water and extracted twice with ethyl acetate. The combined organic layers were washed with brine, solvent was removed and the residue was purified by silica gel chromatography furnishing the desired compound (**17**) as a pale yellow solid (340 mg, 75 %).

**¹H-RMN (200 MHz, CDCl₃)** : 1.13 (s, 9H); 1.26 (s, 9H); 3.95 (m, 2H); 4.00 (s, 3H); 4.40 (m, 2H); 6.81-7.18 (m, 4H), 7.43 (m, 3H); 8.23 (d, J = 8.0 Hz, 2H); 9.01 (s, 2H) ppm.

### Example 12

**4-*tert*-butyl-*N*-[6-(2-hydroxyethoxy)-5-(2-methoxy-phenoxy)-[2,2']bipyrimidinyl-4-yl]-benzenesulfonamide (Bosentan, 1)**

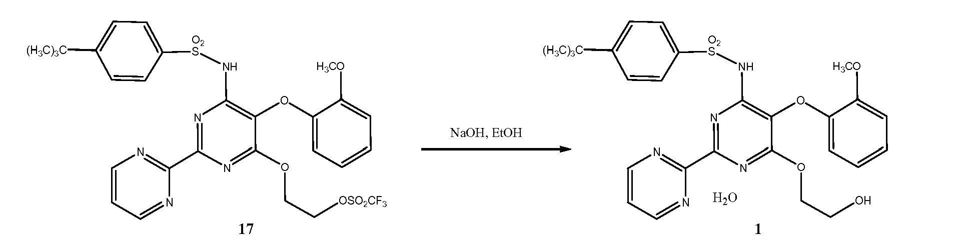

Compound (**17**) (1.0 g, 1.45 mmol) was dissolved in 10 mL of EtOH and then solid NaOH (175 mg, 4.4 mmol) was added. The reaction mixture was stirred at room temperature for 1 h. Then, CH₂Cl₂ and H₂O were added, the pH was adjusted to 3-3.5 with 3 M HCl and the organic layer was separated and dried. After solvent was evaporated, the residue thus obtained was purified by recrystallization in hot H₂O / EtOH affording pure bosentan monohydrate (**1**) as a white solid (743 mg, 90 %).

**¹H-RMN (200 MHz, d₆-DMSO)** : 1.29 (s, 9H); 3.85 (m, 2H); 3.93 (s, 3H); 4.59 (m, 2H); 6.81-7.18 (m, 4H), 7.43 (d, J = 8.0 Hz, 3H); 8.45 (d, J = 8.0 Hz, 2H); 9.01 (s, 2H) ppm.

## Claims

1. A process for the preparation of Bosentan or a pharmaceutically salt thereof which comprises a coupling reaction between a pyrimidine compound of formula **I:** 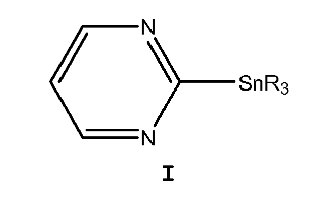 wherein R is a linear or branched C₁-C₅ alkyl
and a sulfonamide compound of formula **II** 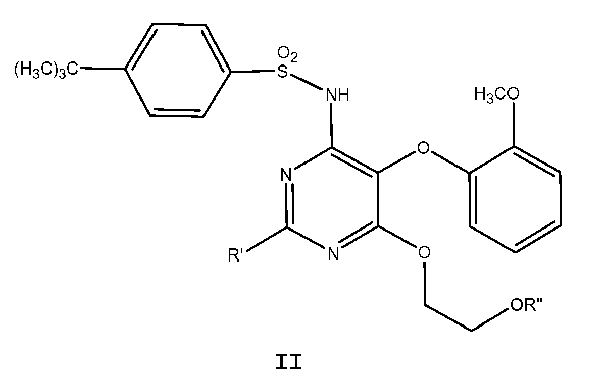 wherein R' is a sulfonyloxy group and R" is selected from hydrogen, a sulfonyl group or a protective groups of alcohols
in the presence of a palladium catalyst and in a solvent medium and, if required, removing the sulfonyloxy group or the protective group of alcohols.

2. The process according to claim 1 wherein the sulfonyloxy group R' in compound of formula **II** is triflate.

3. The process according to claim 1 wherein R in compound of formula **I** is butyl, R' in compound of formula **II** is triflate and R" in compound of formula **II** is selected from hydrogen, a sulfonyl group or a protective group of alcohols.

4. The process according to claim 1, **characterised in that** being the reaction performed in homogeneous conditions, the palladium catalyst is selected from Pd (PPh₃)₄, PdCl₂ (PPh₃)₂, Pd(AcO)₂, Pd₂(dba)₃, PdCl₂(dppf), PdCl₂(CH₃CN)₂ and Pd[P^{t}Bu₃]₂, wherein Ac is acetate, Ph is phenyl, dba is dibenzylideneacetone, dppf is 1,1'-bis(diphenylphosphino)ferrocene and ^{t}Bu is *tert*-butyl.

5. The process according to claim 4, **characterised in that** being the palladium catalyst selected from Pd(AcO)₂ and Pd₂(dba)₃ the coupling reaction is carried out in the presence of a ligand.

6. The process according to claim 5, **characterised in that** said ligand is of the phosphine type, preferably triphenylphosphine or tri-tert-butylphosphine.

7. The process according to claim 1, **characterized in that** the solvent medium is water, an organic solvent or mixtures thereof.

8. The process according to claim 7, **characterized in that** the organic solvent is selected from THF, DME, DEM, toluene, xylene, methanol, ethanol, propanol, dioxane, acetonitrile, DMF and DMA.

9. The process according to claim 1, **characterised in that** the solvent medium further comprises an organic base selected from tertiary amines, like triethyl amine (TEA) or N,N-diisopropylethylamine (DIPEA), or acetates; or other ammonia compounds in which one or more hydrogen have been substituted by alkyl or aryl radicals such as ethyl, isopropyl, benzyl, phenyl or pyridine.

10. A sulfonamide compound of formula **II** or salts thereof: 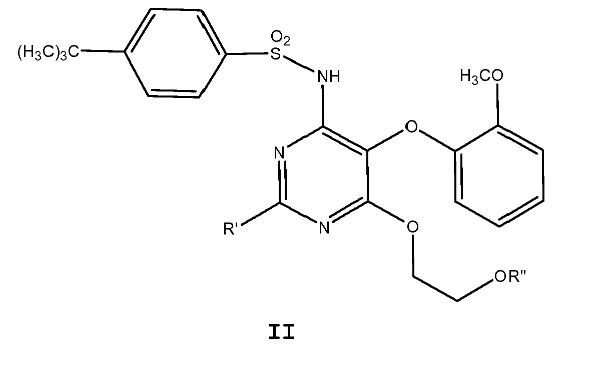 wherein R' is a sulfonyloxy group and R" is selected from hydrogen, a sulfonyl group and a protective groups of alcohols.

11. A process for the preparation of a sulfonamide compound of formula **II** according to claim 10, which comprises the following steps:
a) replacement of the methylsulfonyl group of compound of formula **III** by an hydroxyl group to yield a compound of formula **IV** 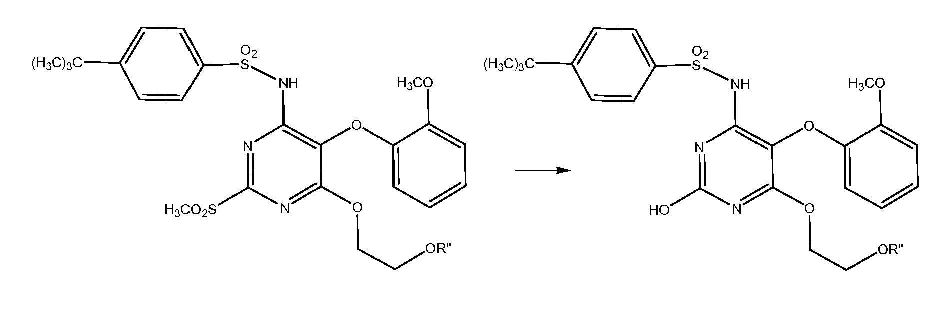 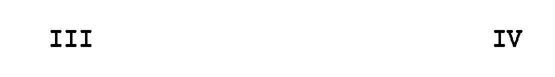 wherein R" is hydrogen or a sulfonyl group or a protective group of alcohols
b) transformation of the hydroxyl group of compound **IV** to a substituted or unsubstituted alkyl or aryl sulfonyloxy group and,
c) if required, removing the sulfonyl group or the protective group of alcohols, R", of compound of formula **IV**

12. A sulfonamide intermediate of formula **IV** 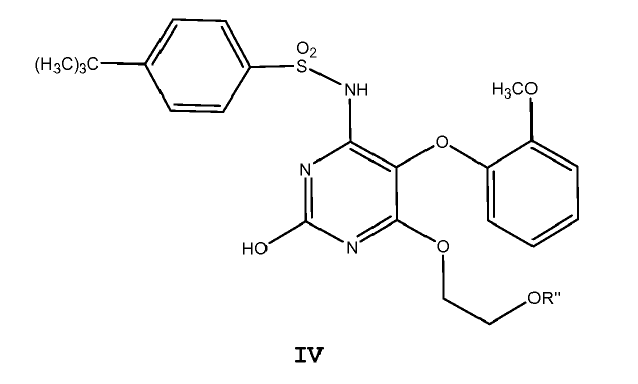 wherein R" is selected from hydrogen, a sulfonyl group and a protective group of alcohols.

13. Use of a sulfonamide intermediate of formula **II** as defined in claim 10 for the preparation of Bosentan.

14. Use of a sulfonamide intermediate of formula **IV** as defined in claim 12 for the preparation of Bosentan.
